(19) Europäisches Patentamt

**European Patent Office**

**Office européen des brevets**

(11) Veröffentlichungsnummer: **0 291 495 B1**

(12) **EUROPÄISCHE PATENTSCHRIFT**

(45) Veröffentlichungstag der Patentschrift: **11.11.92**

(21) Anmeldenummer: **88890109.7**

(22) Anmeldetag: **05.05.88**

(51) Int. Cl.⁵: **D21C 11/00**, C07D 307/50, D21C 3/22

(54) Kombiniertes Verfahren zur thermischen und chemischen Behandlung von lignocellulosehaltiger Biomasse und zur Gewinnung von Furfural.

(30) Priorität: **12.05.87 AT 1189/87**

(43) Veröffentlichungstag der Anmeldung:
**17.11.88 Patentblatt 88/46**

(45) Bekanntmachung des Hinweises auf die Patenterteilung:
**11.11.92 Patentblatt 92/46**

(84) Benannte Vertragsstaaten:
**CH DE ES FR IT LI SE**

(56) Entgegenhaltungen:
**EP-A- 0 038 317**
**EP-A- 0 124 507**
**DE-A- 2 610 682**

(73) Patentinhaber: **VOEST-ALPINE INDUSTRIEAN-LAGENBAU GmbH**
**Turmstrasse 44**
**A-4020 Linz(AT)**

(72) Erfinder: **Avignon, Gerard, Ing.Mag.**
**6, rue J.F.Blade**
**F-47000 Agen(FR)**
Erfinder: **Jaeggle, Wolfgang, Dipl.-Ing.**
**Mörikestrasse 48**
**W-7981 Bodnegg(DE)**
Erfinder: **Steinmüller, Horst, Dipl. Ing.**
**Weberstr. 16**
**A-4060 Leonding(AT)**
Erfinder: **Lackner, Karl**
**Weigunystr. 8a**
**A 4040 Linz(AT)**

(74) Vertreter: **Wolfram, Gustav, Dipl.-Ing.**
**Patentanwälte Sonn, Pawloy, Weinzinger &**
**Wolfram, Riemergasse 14**
**A-1010 Wien(AT)**

**Beschreibung**

Die Erfindung betrifft ein kombiniertes Verfahren zur thermischen und chemischen Behandlung von lignocellulosehaltiger Biomasse und zur Gewinnung von Furfural, wobei die Biomasse einem Kocher chargenweise zugeführt wird, im Kocher unter Beifügung von Kochflüssigkeit erhitzt wird und Zellstoff oder vorbehandelte Biomasse dem Kocher chargenweise entnommen wird, sowie eine Anlage zur Durchführung des Verfahrens.

Ein Verfahren dieser Art ist beispielsweise aus der DE-A - 34 35 451 und aus der EP-B - 0 038 317 bekannt. In der DE-A - 34 35 451 ist zur Herstellung von Zellstoff und Furfural ein zweistufiges Verfahren mit Vorhydrolyse und anschließendem chemischen Aufschluß beschrieben. Dabei wird das aufzuschließende Material in Gegenwart einer verdünnten Säure bei Temperaturen unter 150°C so lange erhitzt, bis die enthaltenen Pentosane zu Pentosen hydrolysiert sind. Danach wird ein Aufschlußmittel zugeführt und die Temperatur schnell auf über 160°C gesteigert und dort so lange gehalten, bis der Aufschluß beendet ist. Dabei wird das sich aus den Pentosen bildende Furfural abgeführt.

In der EP-B - 0 038 317 ist ein Verfahren zur Gewinnung von Furfural und anderen organischen Verbindungen aus sauren Hydrolysaten von Pflanzen, insbesondere Sulfitablaugen, beschrieben. Dabei werden die in den Ablaugen enthaltenen Pentosen durch Erhitzen in einem Reaktor auf Temperaturen bis zu 300°C zu Furfural dehydratisiert.

Der Reaktionsablauf der Bildung von Furfural aus in lignocellulosehaltiger Biomasse enthaltenen Pentosanen umfaßt zwei durch Zugabe von Säuren begünstigte Reaktionsschritte. Der erste besteht in einer Wasseraufnahme, wobei die Pentosane durch Hydrolyse in Pentosen übergeführt werden, und der zweite in einer Dehydratisierung, wodurch die Pentosen in Furfural umgewandelt werden. Beim Verfahren nach der DE-A - 34 35 451 laufen diese beiden Reaktionsschritte während des chemischen Aufschlusses der Biomasse ab. Durch die hierbei drastischen Reaktionsbedingungen kommt es aber teilweise zur Bildung von Kondensationsprodukten aus Furfural und zu Zersetzungsreaktionen der Pentosane. Die gleichen Nachteile weist auch das Verfahren nach der EP-B - 0 038 317 auf, bei dem Furfural erst nach dem Aufschluß aus der Ablauge gebildet wird. Bei beiden Verfahren ergibt sich daher gegenüber der theoretisch maximal möglichen Furfuralausbeute eine nur stark verminderte Ausbeute durch Bildung von Kondensationsprodukten, die sich im Falle der Herstellung von gebleichtem Sulfitzellstoff auch auf diesen nachteilig auswirken.

Die Erfindung bezweckt die Vermeidung dieser Schwierigkeiten und Nachteile und stellt sich die Aufgabe, ein kombiniertes Verfahren der eingangs beschriebenen Art sowie eine Anlage zur Durchführung des Verfahrens zu schaffen, durch die die Bildung von Folgeprodukten aus Pentosen im Kochprozeß selbst verhindert werden soll, so daß die Ausbeute an Furfural erhöht wird, und durch die bei der Herstellung von gebleichtem Sulfitzellstoff nur ein geringer Bleichmitteleinsatz erforderlich ist.

Diese Aufgabe wird erfindungsgemäß dadurch gelöst, daß dem Kocher während der Aufheizphase, gegebenenfalls auch während der Kochphase, Kochflüssigkeit entnommen und einer Furfuralerzeugungsanlage zugeführt wird und daß die von Pentosen bzw. Furfural zumindest größtenteils befreite Kochflüssigkeit in den Kocher rückgeführt wird, wobei die Kochflüssigkeit während der Kochphase vorwiegend nur über einen Wärmetauscher geleitet wird.

Die im Vergleich zur späteren Aufschlußphase milden Reaktionsbedingungen der Aufheizphase, insbesondere deren niedrige Temperatur, gestatten einerseits die Hydrolyse der Pentosane zu Pentosen, und verhindern andererseits die Weiterreaktion zu Furfural und dessen Kondensationsprodukten. Dadurch wird erstens die Ausbeute an Pentosen bzw. Furfural stark erhöht und zweitens bei der Herstellung von Zellstoff dessen Reinheit verbessert, wodurch mit einem geringeren Bleichmitteleinsatz in der weiteren Verarbeitung das Auslangen gefunden wird. Die Rückführung der Kochflüssigkeit ermöglicht eine kontinuierliche Abtrennung der Pentosen, ohne dabei die Aufheizphase unterbrechen zu müssen. Außerdem wird ein Verarmen des Kochers an Kochflüssigkeit vermieden.

Gemäß einer bevorzugten Ausführungsform werden in der Furfuralerzeugungsanlage die in der Kochflüssigkeit enthaltenen Pentosen zu Furfural dehydratisiert, das erhaltene Substrat einer Wasserdampfdestillation unterworfen, die zurückbleibende Kochflüssigkeit in den Kocher rückgeführt und die kondensierten Brüden einer einoder mehrstufigen Destillation zur Abtrennung des Furfurals unterworfen.

Zweckmäßig ist auch, wenn die bei der ein- oder mehrstufigen Destillation anfallende Flüssigkeit in den Kocher rückgeführt wird.

Vorteilhaft wird bei der Herstellung von Zellstoff das bei der Wasserdampfdestillation anfallende Schwefeldioxid in den Kocher rückgeführt.

Dadurch wird ein Verarmen der im Kocher befindlichen Kochflüssigkeit an Schwefeldioxid vermieden, und es erübrigt sich daher ein ständiges Ergänzen jener Schwefeldioxidmenge, die mit der Kochflüssigkeit in die Furfuralerzeugungsanlage gelangt.

Bevorzugt wird die Pentosen-enthaltende Kochflüssigkeit durch die Furfuralerzeugungsanlage mit einer Gesamtverweilzeit in einem Bereich zwischen 10 und 60 min, vorzugsweise zwischen 10 und 25 min, geführt, wobei die Kochflüssigkeit auf eine Temperatur zwischen 130 und 180°C, vorzugsweise zwischen 150 und 180°C, aufgeheizt und in diesem Bereich gehalten wird.

Naturgemäß schwankt der Gehalt der Kochflüssigkeit an Pentosen während der Aufheizphase. Durch Festlegen einer Gesamtverweilzeit in der Furfuralerzeugungsanlage bei einer bestimmten Temperatur kann die Reaktionsbedingung der Dehydratisierung dem jeweiligen Gehalt an Pentosen angepaßt und damit die Furfuralausbeute verbessert werden.

Zur optimalen Anpassung der Reaktionsbedingungen an den zu verarbeitenden Rohstoff und an die Betriebsweise des Kochers kann es auch vorteilhaft sein, die Kochflüssigkeit dem Kocher im Anschluß an die Aufheizphase der nachfolgenden Kochphase zu entnehmen.

Eine Anlage zur Durchführung des Verfahrens umfaßt einen Kocher, in den eine Zuführungsleitung für Lignocellulosehaltige Biomasse einmündet und an den eine Ableitung für im Kocher thermisch behandelte Biomasse sowie eine zu einem Wärmetauscher führende Zweigleitung angeschlossen ist, von dem eine in den Kocher mündende Rückleitung für die Kochflüssigkeit ausgeht, und eine, einen Rohrreaktor, eine Reaktionskolonne und eine nachfolgende Destillationseinrichtung aufweisende Furfuralerzeugungsanlage, deren Rohrreaktor mit einer Zulaufleitung und deren Reaktionskolonne mit einer Rücklaufleitung mit dem Kocher leitungsmäßig verbindbar sind.

Gemäß einer zweckmäßigen Ausführungsform ist die Destillationseinrichtung zur Rückführung von vom Furfural abgetrennter Flüssigkeit mit dem Kocher leitungsmäßig verbindbar. Damit kann die von Furfural befreite Kochflüssigkeit wieder in den Kocher zurückgeleitet werden.

Gemäß einer weiteren zweckmäßigen Ausführungsform mündet eine Brüden-Ableitung der Reaktionskolonne in einen Sammelbehälter, der mit der Destillationseinrichtung verbunden ist, wobei der Sammelbehälter einen Gasraum aufweist, der leitungsmäßig mit dem Kocher verbindbar ist.

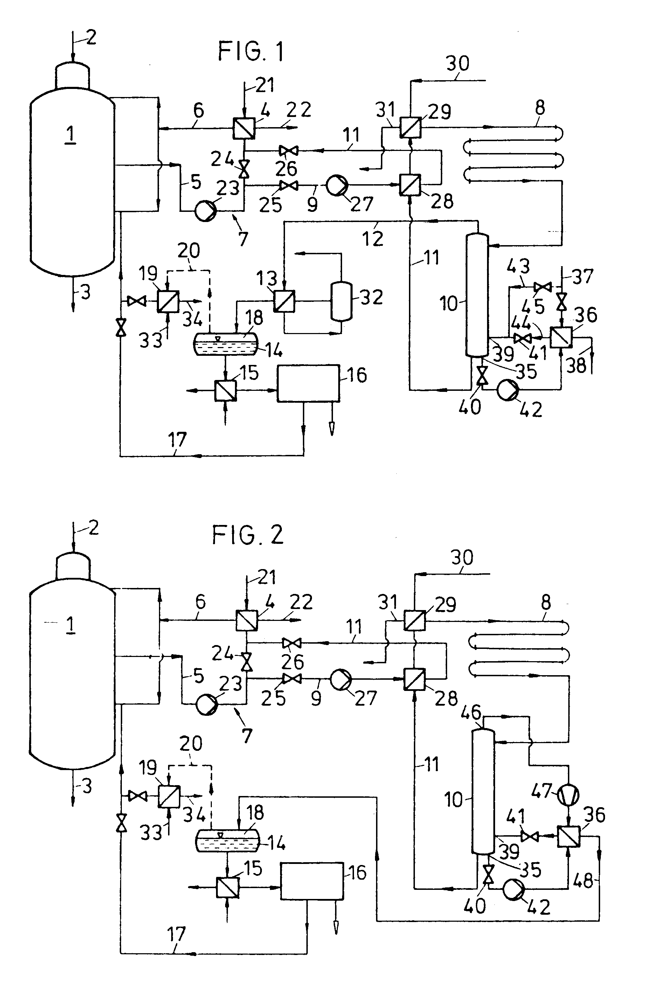

Das erfindungsgemäße Verfahren und eine Anlage zur Durchführung des Verfahrens sind nachfolgend anhand der Fig. 1 und 2 sowie zweier Ausführungsbeispiele näher erläutert. Beide Figuren zeigen ein Blockschema der Anlage nach je einer Ausführungsförm.

Gemäß Fig. 1 mündet in einen Kocher 1 eine Zuführungsleitung 2 zur Beschickung des Kochers mit Kochflüssigkeit und Biomasse. Weiters sind am Kocher 1 eine Ableitung 3 zur Entnahme von vorbehandelter Biomasse bzw. Sulfitzellstoff sowie eine zu einem für die Aufheizung der Kochflüssigkeit vorgesehenen Wärmetauscher 4 führende Zweigleitung 5 angeschlossen. Vom Wärmetauscher 4 geht eine Rückleitung 6 für die Kochflüssigkeit aus, die in den Kocher 1 mündet und somit einen Heizkreislauf 7 schließt. An die Zweigleitung 5 sind bypass-artig ein Rohrreaktor 8 über eine Zulaufleitung 9 und eine zum Rohrreaktor 8 in Serie geschaltete Reaktionskolonne 10 über eine Rücklaufleitung 11 angeschlossen. Von der Reaktionskolonne führt eine Brüden-Ableitung 12 über einen Wärmetauscher 13, einen Sammelbehälter 14 und über einen weiteren Wärmetauscher 15 zu einer Destillationseinrichtung 16, die über eine Leitung 17 für den Destillationsrückstand mit dem Kocher 1 verbunden ist. Wird die oben beschriebene Anlage zur Herstellung von Sulfitzellstoff eingesetzt, ist zweckmäßig zusätzlich der Gasraum 18 des Sammelbehälters 14 über einen Wärmetauscher 19 mit dem Kocher 1 über eine in den Figuren mit strichlierten Linien dargestellte Leitung 20 verbindbar.

Das erfindungsgemäße Verfahren wird wie folgt durchgeführt:
Nachdem der Kocher 1 über die Zuführungsleitung 2 mit lignocellulosehaltiger Biomasse und Kochflüssigkeit beschickt wurde, wird mit der Aufheizung begonnen. Dazu wird über die Zweigleitung 5 Kochlauge aus dem Kocher 1 abgezogen und in den mit Hochdruckdampf (veranschaulicht durch die Pfeile 21, 22) versorgten Wärmetauscher 4 geleitet, dort erhitzt und über die Rückleitung 6 wieder in den Kocher 1 zurückgeleitet. Die Umwälzung der Kochlauge wird von einer in der Zweigleitung 5 vorgesehenen Pumpe 23 besorgt. Zwischen der Abzweigung der Zulaufleitung 9 und der Einmündung der Rücklaufleitung 11 ist in der Zweigleitung ein Ventil 24 vorgesehen, das bei der direkten Umwälzung der Kochflüssigkeit über den Wärmetauscher 4 geöffnet ist, wogegen jeweils ein in der Zulaufleitung 9 und in der Rücklaufleitung 11 befindliches Ventil 25, 26 geschlossen ist.

Sobald im Kocher Pentosane zu Pentosen hydrolysiert und dabei von der Kochflüssigkeit gelöst werden, wird das in der Zweigleitung eingebaute Ventil 24 geschlossen und werden die in der Zu- und Rücklaufleitung vorgesehenen Ventile 25 und 26 geöffnet, so daß Kochflüssigkeit über die Zulaufleitung 9 in den Rohrreaktor 8 mit Hilfe einer Pumpe 27, die in der Zulaufleitung 9 angeordnet ist, gepumpt wird. Auf diesem Weg wird die Kochflüssigkeit in zwei ebenfalls in der Zulaufleitung vorgesehenen Wärmetauschern 28 und 29, von denen einer (28) mit über die Rücklaufleitung 11 rücklaufender Kochflüssigkeit und der andere (29) mit Hochdruckdampf (veranschaulicht durch die Pfeile 30 und 31) versorgt werden, geleitet und dabei auf die für die Dehydratisierung der Pentosen zu Furfural notwendige Temperatur gebracht. Dieser Reaktionsschritt erfolgt größtenteils im Rohrreaktor 8 und teilweise auch noch in der Reaktionskolonne 10, in die die Kochflüssigkeit anschließend geleitet wird. In der Reaktionskolonne 10 wird mit Wasserdampf das gebildete Furfural aus der Kochflüssigkeit ausgetrieben, d.h. abgestrippt, über die Brüden-Ableitung 12 in den Wärmetauscher 13 geleitet, dort unter Bildung von Sekundärdampf kondensiert und die dabei gebildete wässerige Furfurallösung im Sammelbehälter 14 aufgefangen. Der sich im Wärmetauscher 13 bildende Sekundärdampf wird in einem Kühler 32 kondensiert und dem Wärmetauscher 13 wieder zugeführt.

Die von Furfural befreite und am unteren Ende der Reaktionskolonne 10 abgezogene Kochflüssigkeit wird mittels der Rückleitung 11 während der Aufheizphase des Kochers 1 kontinuierlich über die Wärmetauscher 28 und 4 in den Kocher rückgeführt.

Die Zuschaltung der Furfuralerzeugungsanlage erfolgt so lange, wie die Pentosenkonzentration in der Kochflüssigkeit einen bestimmten Grenzwert nicht unterschreitet. Danach erfolgt die thermische Vorbehandlung unter Leitung der Kochflüssigkeit über den Heizkreislauf 7, bis die lignocellulosehaltige Biomasse fertig behandelt ist, worauf der Kocher entleert und neu chargiert wird.

Wird die Anlage zur Herstellung von Sulfitzellstoff betrieben, so fällt im Gasraum 18 des Sammelbehälters 14 Schwefeldioxid an, das im Wärmetauscher 19, der mit Hochdruckdampf, veranschaulicht durch die Pfeile 33 und 34, versorgt wird, erhitzt wird und anschließend wieder in den Kocher 1 rückgeleitet wird.

Die wässerige Furfurallösung im Sammelbehälter 14 wird im Wärmetauscher 15 unter Bildung von Sekundärdampf gekühlt und der Furfuraldestillationseinrichtung 16 zugeführt, in der in einer oder mehreren Stufen Furfural gewonnen wird. Der dabei anfallende Destillationsrückstand wird über die Leitung 17 wieder in den Kocher 1 zurückgeleitet.

Die Wärmeversorgung der Rekationskolonne 10 kann sowohl bei der Herstellung von Zellstoff als auch zur Herstellung von vorbehandelter Biomasse zweckmäßigerweise in zwei Varianten erfolgen:
Gemäß Fig. 1 wird die Kochflüssigkeit der Reaktionskolonne am unteren Ende 35 derselben entnommen, im Wärmetauscher 36 mit Hochdruckdampf (veranschaulicht durch die Pfeile 37, 38) erhitzt und wieder in die Reaktionskolonne 10 nahe deren unterem Ende 39 zurückgeleitet. Die Umwälzung wird mittels Ventile 40 und 41 und einer Pumpe 42 gesteuert. Von der Hochdruckdampfzuleitung 37 führt eine Hochdruckdampf-Zweigleitung 43 in die vom Wärmetauscher 36 ausgehende Rückführung 44, wobei in der HochdruckdampfZweigleitung 43 ein Regelventil 45 vorgesehen ist. Durch ein Zusammenspiel der Ventile 40, 41 und 45 kann Heißdampf sowohl zur Wärmeversorgung des Wärmetauschers 36 als auch zur Einspeisung in die Reaktionskolonne 10 herangezogen werden.

Gemäß Fig. 2 werden die die Reaktionskolonne 10 am oberen Ende 46 verlassenden Brüden in einem Kompressor 47 verdichtet und zur Wärmeversorgung des Wärmetauschers 36 verwendet. Die im Wärmetauscher 36 kondensierten Brüden werden danach über eine Leitung 48 in den Sammelbehälter 14 geleitet und, wie oben beschrieben, aufgearbeitet.

Ausführungsbeispiel 1:

Aus Buchenholz wird nach dem Magnesiumbisulfitverfahren durch chargenweise Kochung Zellstoff gewonnen.
Rohstoffanalyse des Buchenholzes:

| | |
|--------|---------|
| Xylan | 24,2 % |
| Glukan | 42,6 % |
| Lignin | 26,4 % |

In den Kocher 1, der ein Volumen von 200 m$^3$ aufweist, werden 36 t atro Buchenholz eingesetzt. Der Hydromodul beträgt 1 : 3,5. Der Kocher 1 wird wie oben beschrieben über den Heizkreislauf 7 mittels Dampf durch Kochflüssigkeitszirkulation aufgeheizt. Bei Erreichen einer Temperatur im Kocher von ca. 120°C (nach ca. 3 Stunden) sind ca. 60 % der Pentosen freigesetzt, worauf Kochflüssigkeit aus dem Heizkreislauf 7 des Kochers 1 abgezweigt, mit der schon von Pentosen befreiten Kochflüssigkeit durch indirekten Wärmetausch vorerwärmt und danach auf eine Temperatur von 160 bis 165°C weitererhitzt wird.

Die Verweilzeit im Rohrreaktor 8 ist in Abhängigkeit der Pentosekonzentration über die Durchflußmenge geregelt. Die Gesamtverweilzeit im System Rohrreaktor 8 - Reaktionskolonne 10 beträgt 15 min. Die Brüden, die der unter Druck stehenden Reaktionskolonne 10 entweichen, enthalten das gesamte freie SO$_2$ der Kochlauge und das gebildete Furfural. Diese Brüden werden bei gleichzeitiger Herstellung von Sekundärdampf kondensiert, das enthaltene SO$_2$ sofort abgetrennt und in Gasphase über die Leitung 20 unter Druck dem Kocher 1 wieder zugeführt, damit der SO$_2$-Gehalt der Kochlauge konstant bleibt. Die Furfural-Destillation wird in zwei Stufen durchgeführt.

Sinkt der Gehalt der Pentosenkonzentration auf unter 5 g/l, wird Kochflüssigkeit nicht mehr über die Furfuralerzeugungsanlage, sondern über den Heizkreislauf 7 geführt. Die in diesem Prozeß aus dem angegebenen Einsatzmaterial erzeugte Furfuralmenge beträgt 4500 kg.

Ausführungsbeispiel 2:

Aus Zuckerhirsebagasse wird durch thermische Behandlung Furfural und vorbehandelte Biomasse für eine nachfolgende enzymatische Hydrolyse gewonnen.

Nach Erreichung der Kochtemperatur von ca. 150°C wird begonnen, dem Kocher 1 laufend die pentosehaltige Kochflüssigkeit abzuziehen, bei gleichzeitiger Rückführung der von Pentosen weitgehend befreiten Kochlfüssigkeit aus der Furfuralerzeugungsanlage. Da die Umsetzung der Pentosen zu Furfural in mineralsaurem Milieu effizienter durchzuführen ist als durch die Säurekatalyse der organischen Säuren aus dem Rohstoff, können von Anfang an der Kochlauge etwa 0,5 bis 2,0 % Schwefelsäure zugesetzt werden. Die Gesamtverweilzeit im System Rohrreaktor und Reaktionskolonne beträgt 15 min.

Auf diese Art können bei einem Pentoseumsetzungsgrad im Rohrreaktor 8 von 80 % und bei einer Furfuralausbeute von ebenfalls 80 % etwa 67 kg Fufural pro Tonne Zuckerhirsebagasse gewonnen werden. Die vorbehandelte Biomasse wird anschließend auf mindestens 200°C erhitzt, um in einer nachfolgenden enzymatischen Hydrolyse eine maximale Glukoseausbeute zu gewährleisten.

**Patentansprüche**

1. Kombiniertes Verfahren zur thermischen und chemischen Behandlung von lignocellulosehaltiger Biomasse und zur Gewinnung von Furfural, wobei die Biomasse einem Kocher (1) chargenweise zugeführt wird, im Kocher (1) unter Beifügung von Kochflüssigkeit erhitzt wird und Zellstoff oder vorbehandelte Biomasse dem Kocher (1) chargenweise entnommen wird, dadurch gekennzeichnet, daß dem Kocher (1) während der Aufheizphase, gegebenenfalls auch während der Kochphase, Kochflüssigkeit entnommen und einer Furfuralerzeugungsanlage (8, 10, 16) zugeführt wird und daß die von Pentosen bzw. Furfural zumindest größtenteils befreite Kochflüssigkeit in den Kocher (1) rückgeführt wird, wobei die Kochflüssigkeit während der Kochphase vorwiegend nur über einen Wärmetauscher (4) geleitet wird.

2. Verfahren nach Anspruch 1, dadurch gekennzeichnet, daß in der Furfuralerzeugungsanlage (8, 10, 16) die in der Kochflüssigkeit enthaltenen Pentosen zu Furfural dehydratisiert, das erhaltene Substrat einer Wasserdampfdestillation unterworfen, die zurückbleibende Kochflüssigkeit in den Kocher (1) rückgeführt und die kondensierten Brüden einer einoder mehrstufigen Destillation zur Abtrennung des Furfurals unterworfen werden.

3. Verfahren nach Anspruch 2, dadurch gekennzeichnet, daß die bei der ein- oder mehrstufigen Destillation anfallende Flüssigkeit in den Kocher (1) rückgeführt wird.

4. Verfahren nach Anspruch 2, dadurch gekennzeichnet, daß bei der Herstellung von Zellstoff das bei der Wasserdampfdestillation anfallende Schwefeldioxid in den Kocher (1) rückgeführt wird.

5. Verfahren nach einem oder mehreren der Ansprüche 1 bis 4, dadurch gekennzeichnet, daß die Pentosen-enthaltende Kochflüssigkeit durch die Furfuralerzeugungsanlage (8, 10, 16) mit einer Gesamtverweilzeit in einem Bereich zwischen 10 und 60 min, vorzugsweise zwischen 10 und 25 min, geführt wird, wobei die Kochflüssigkeit auf eine Temperatur zwischen 130 und 180°C, vorzugsweise zwischen 150 und 180°C, aufgeheizt und in diesem Bereich gehalten wird.

6. Verfahren nach einem oder mehreren der Ansprüche 1 bis 5, dadurch gekennzeichnet, daß dem Kocher (1) im Anschluß an die Aufheizphase auch während der nachfolgenden Kochphase Kochflüssigkeit entnommen wird.

7. Anlage zur Durchführung des Verfahrens nach einem oder mehreren der Ansprüche 1 bis 6, umfassend einen Kocher (1), in den eine Zuführungsleitung (2) für Lignocellulose-haltige Biomasse einmündet und an den eine Ableitung (3) für im Kocher thermisch behandelte Biomasse sowie eine zu einem Wärmetauscher (4) führende Zweigleitung (5) angeschlossen ist, von dem eine in den Kocher

EP 0 291 495 B1

mündende Rückleitung (6) für die Kochflüssigkeit ausgeht, und eine, einen Rohrreaktor (8), eine Reaktionskolonne (10) und eine nachfolgende Destillationseinrichtung (16) aufweisende Furfuralerzeugungsanlage, deren Rohrreaktor (8) mit einer Zulaufleitung (9) und deren Reaktionskolonne (10) mit einer Rücklaufleitung (11) mit dem Kocher (1) leitungsmäßig verbindbar sind.

8.  Anlage nach Anspruch 7, dadurch gekennzeichnet, daß die Destillationseinrichtung (16) zur Rückführung von vom Furfural abgetrennter Flüssigkeit mit dem Kocher (1) leitungsmäßig verbindbar ist.

9.  Anlage nach Anspruch 7 oder 8, dadurch gekennzeichnet, daß eine Brüden-Ableitung (12) der Reaktionskolonne (10) in einen Sammelbehälter (14) mündet, der mit der Destillationseinrichtung (16) verbunden ist, wobei der Sammelbehälter (14) einen Gasraum (18) aufweist, der leitungsmäßig mit dem Kocher (1) verbindbar ist.

**Claims**

1.  A combined process for thermally and chemically treating lignocellulose-containing biomass and for recovering furfural, wherein the biomass is supplied to a cooker (1) in batches, is heated in the cooker (1) under addition of a cooking liquor, and cellulose or pre-treated biomass is withdrawn from the cooker (1) in batches, characterised in that cooking liquor is withdrawn from the cooker (1) during the heating phase, possibly also during the cooking phase, and is supplied to a furfural production plant (8, 10, 16), and that the cooking liquor at least substantially freed from pentoses and furfural, respectively, is returned into the cooker (1), wherein during the cooking phase the cooking liquor is mainly guided through a heat exchanger (4).

2.  A process according to claim 1, characterised in that in the furfural production plant (8, 10, 16) the pentoses contained in the cooking liquor are dehydrated to furfural, the substrate obtained is subjected to a steam distillation, the remaining cooking liquor is returned into the cooker (1) and the condensed exhaust vapors are subjected to a single- or multi-stage distillation for separating the furfural.

3.  A process according to claim 2, characterised in that the liquid incurred in the single- or multi-stage distillation is returned into the cooker (1).

4.  A process according to claim 2, characterised in that sulphur dioxide incurred in the steam distillation at the production of cellulose is returned into the cooker (1).

5.  A process according to one or several of claims 1 to 4, characterised in that the pentoses-containing cooking liquor is guided through the furfural production plant (8, 10, 16) at a total residence time in a range of between 10 and 60 min, preferably between 10 and 25 min, wherein the cooking liquor is heated to a temperature of between 130 and 180°C, preferably between 150 and 180°C, and maintained in that range.

6.  A process according to one or several of claims 1 to 5, characterised in that cooking liquor is also withdrawn from the cooker (1) subsequent to the heating phase, during the subsequent cooking phase.

7.  A plant for carrying out the process according to one or several of claims 1 to 6, comprising a cooker (1), into which a feed duct (2) for lignocellulose-containing biomass enters and to which a discharge duct (3) for biomass thermally treated in the cooker as well as a branch duct (5) leading to a heat exchanger (4) are connected, from which there departs a cooking-liquor return duct (6) entering into the cooker, and a furfural production plant comprising a pipe reactor (8), a reaction column (10) and a consecutive distillation device (16), whose pipe reactor (8) is capable of being flow-connected with the cooker (1) by a supply duct (9) and whose reaction column (10) is capable of being flow-connected with the cooker (1) by a return duct (11).

8.  A plant according to claim 7, characterised in that the distillation device (16) for returning liquid separated from furfural is capable of being flow-connected with the cooker (1).

9.  A plant according to claim 7 or 8, characterised in that an exhaust vapor discharge duct (12) of the reaction column (10) enters into a collecting basin (14), which is connected with the distillation device (16), wherein the collecting basin (14) comprises a gas space (18) capable of being flow-connected with the cooker (1).

**Revendications**

1. Procédé combiné de traitement thermique et chimique d'une biomasse lignocellulosique et d'obtention de furfural, la biomasse étant amenée par lots à un appareil de cuisson (1), chauffée dans l'appareil de cuisson (1) cependant qu'est ajouté un liquide de cuisson et la cellulose ou biomasse prétraitée étant prélevée par lots dans l'appareil de cuisson (1), caractérisé en ce que du liquide de cuisson est prélevé dans l'appareil de cuisson (1) pendant la phase de montée en température, le cas échéant également pendant la phase de cuisson, et amené à une installation de production de furfural (8, 10, 16) et en ce que le liquide de cuisson débarrassé du moins en majeure partie des pentoses ou du furfural est recyclé à l'appareil de cuisson (1), le liquide de cuisson ne passant essentiellement pendant la phase de cuisson que sur un échangeur de chaleur (4).

2. Procédé selon la revendication 1, caractérisé en ce que les pentoses contenus dans le liquide de cuisson sont déshydratés en furfural dans l'installation de production de furfural (8, 10, 16), que le substrat obtenu est soumis à un entraînement à la vapeur, que le liquide de cuisson résiduel est ramené dans l'appareil de cuisson (1) et que les buées condensées sont soumises à une distillation en une ou plusieurs étapes en vue de la séparation du furfural.

3. Procédé selon la revendication 2, caractérisé en ce que le liquide obtenu lors de la distillation en une ou plusieurs étapes est ramené dans l'appareil de cuisson (1).

4. Procédé selon la revendication 2, caractérisé en ce que le dioxyde de soufre se formant lors de l'entraînement à la vapeur lors de la production de cellulose est ramené dans l'appareil de cuisson (1).

5. Procédé selon une ou plusieurs des revendications 1 à 4, caractérisé en ce que le liquide de cuisson contenant des pentoses est acheminé dans l'installation de production de furfural (8, 10, 16) pour une durée de séjour totale comprise dans une plage de 10 à 60 min, de préférence de 10 à 25 min, le liquide de cuisson étant chauffé à une température comprise entre 130 et 180°C, de préférence entre 150 et 180°C, et maintenu dans cette plage.

6. Procédé selon une ou plusieurs des revendications 1 à 5, caractérisé en ce que du liquide de cuisson est également prélevé dans l'appareil de cuisson (1), à la suite de la phase de montée en température, pendant la phase de cuisson subséquente.

7. Installation de mise en oeuvre du procédé selon une ou plusieurs des revendications 1 à 6, comprenant un appareil de cuisson (1) dans lequel débouche une conduite d'amenée (2) d'une biomasse lignocellulosique et auquel est relié un branchement (3) destiné à la biomasse traitée thermiquement dans l'appareil de cuisson ainsi qu'une conduite dérivée (5) menant à un échangeur de chaleur (4), d'où part une conduite de retour (6) destinée au liquide de cuisson, débouchant dans l'appareil de cuisson et une installation de production de furfural présentant un réacteur tubulaire (8), une colonne de réaction (10) et un dispositif de distillation (16) en aval, dont le réacteur tubulaire (8) peut être relié par une conduite d'amenée (9) et la colonne de réaction (10) par une conduite de retour (11) à l'appareil de cuisson (1).

8. Installation selon la revendication 7, caractérisée en ce que le dispositif de distillation (16) peut être relié par une conduite à l'appareil de cuisson (1) en vue du recyclage du liquide séparé du furfural.

9. Installation selon la revendication 7 ou 8, caractérisée en ce qu'un branchement de buées (12) de la colonne de réaction (10) débouche dans un récipient collecteur (14) relié au dispositif de distillation (16), le récipient collecteur (14) présentant un compartiment de gaz (18) qui peut être relié par une conduite à l'appareil de cuisson (1).

FIG. 1

FIG. 2